(19)

Europäisches Patentamt
European Patent Office
Office européen des brevets

(11) **EP 3 456 250 A1**

(12) **EUROPEAN PATENT APPLICATION**

(43) Date of publication:
**20.03.2019 Bulletin 2019/12**

(51) Int Cl.:
*A61B 5/02* (2006.01)    *A61B 5/021* (2006.01)
*A61B 5/026* (2006.01)    *G06T 7/00* (2017.01)
*A61B 5/055* (2006.01)

(21) Application number: **17191637.2**

(22) Date of filing: **18.09.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Charité - Universitätsmedizin Berlin 10117 Berlin (DE)**

(72) Inventors:
• **Fernandes, Joao Filipe N15 4AS London (GB)**
• **Kühne, Titus 14129 Berin (DE)**

(74) Representative: **Bittner, Thomas L. Boehmert & Boehmert Anwaltspartnerschaft mbB Pettenkoferstrasse 22 80336 München (DE)**

(54) **METHOD AND SYSTEM FOR NON-INVASIVELY DETERMINING PRESSURE DIFFERENCE IN A CARDIOVASCULAR VESSEL**

(57)    The disclosure refers to a method for non-invasively determining a pressure difference in a cardiovascular vessel, comprising providing imaging data indicative of imaging signals detected in a region of interest of a cardiovascular vessel, the region comprising a proximal vessel portion, a distal vessel portion, and a vessel narrowing located between the proximal and the distal vessel; and determining, from the imaging data, a pressure difference between a first location and a second location in the cardiovascular vessel portion, the first location and the second location provided in the proximal and the distal vessel portions, respectively. The determining of the pressure difference comprises: determining, from the imaging data, a preliminary pressure value indicating pressure difference between the first location and the second location; determining, from the imaging data, time dependent velocity data for blood flow in the first location and the second location; determining, from the time dependent velocity data, a velocity and time dependent pressure value indicating a velocity and time dependent pressure difference between the first location and the second location; and determining the pressure difference for the first location and the second location by combining the preliminary pressure value and the velocity dependent pressure value. Further, a system for non-invasively determining a pressure difference in a cardiovascular vessel is provided.

Fig. 1

## Description

The present disclosure refers to a method and a system for non-invasively determining a pressure difference in a cardiovascular vessel.

### Background

In coarctation of the aorta (CoA), clinical guidelines recommend treatment in the presence of relevant pressure difference across the CoA and / or arterial hypertension. Accordingly, reliable non-invasive methods for measuring pressure differences which may also be referred to as pressure gradients are of clinical importance but currently available methods have limited accuracy.

CoA prevails in approximately 3 out of 10 000 births, corresponding to 5-8% of all congenital heart conditions (Baumgartner et al., European Heart Journal, 2010, 31(23): p. 2915-2957). In CoA the LV is exposed to chronic pressure overload. In addition, arterial hypertension can develop and turn into a "persistent" form if left untreated.

To prevent end-organ damage and development of vascular complications, American College of Cardiology (ACC) and American Heart Association (AHA) guidelines recommend intervention of CoA or re-CoA in cases, with peak-to-peak pressure gradient across the CoA exceeding 20 mmHg and / or significant CoA narrowing (Warnes et al., J Am Coll cardiol, 2008, 52(23): p. e143-263). The European Society of Cardiology (ESC) recommend interventional treatment if CoA pressure gradients are higher than 20 mmHg between upper and lower limbs and arterial hypertension (>140 / 90 mmHg) is present. However, these guidelines are not specific in terms of which method shall be used for pressure gradient assessment, and refer cardiac catheterization as the gold standard for CoA evolution at many centres (Baumgartner et al., European Heart Journal, 2010, 31(23): p. 2915-2957).

The guidelines highlight the clinical need for accurately measure pressure gradients in these patients, ideally non-invasively. However, current non-invasive methods are unsatisfactory. Riva-Rocci (RR) provides the pressure differences between peripheral arteries and is affected by anatomic variation, pulse wave velocities and measurement set-up (e.g. cuff-size). Doppler echocardiography applies the Bernoulli simplified equation, which tends to overestimate the CoA pressure gradient, since maximal velocities across CoA increase due to higher wall stiffness. This method might also lead to underestimation if the maximum velocity is not detected in Doppler acquisition field of view. With the implementation of 4-direction velocity encoded MRI (4D-VENC MRI), it became possible to map the relative pressure variation in one vessel (Tyszka et al., Journal of Magnetic Resonance Imaging, 2000, 12(2): p. 321-329). However, relative pressure maps do not take into account the patient-specific time-shift of peak pressures between the ascending and descending aorta, that is dependent on vessel wall compliance and arterial resistance. It was proposed (Riesenkampff et al., JACC: Cardiovascular Imaging, 2014, 7(9): p. 920-926) to conduct manual correction by calibrating standard pressure curves with RR absolute pressure measurements. However, this method is cumbersome and requires assessment of several aortic locations.

### Summary

It is an object of the present disclosure to provide technologies for non-invasively determining a pressure difference in a cardiovascular vessel.

For solving the object, a method and a system for non-invasively determining a pressure difference in a cardiovascular vessel according to the independent claims 1 and 9 are provided. Further embodiments are disclosed in dependent claims.

According to an aspect, a method for non-invasively determining an absolute pressure difference in a cardiovascular vessel is provided, comprising: providing imaging data indicative of imaging signals detected for a region of a cardiovascular vessel, the region comprising an proximal vessel portion, a distal vessel portion, and a vessel narrowing located between the proximal and the distal vessel portion; and determining, from the imaging data, a pressure difference for a first location and a second location in the cardiovascular vessel, the first location and the second location provided in the proximal and the distal vessel portions, respectively.

According to another aspect, a system for non-invasively determining a pressure difference in a cardiovascular vessel, the system comprising one or more processors configured to: receive imaging data indicative of imaging signals detected in a region of interest of a cardiovascular vessel, the region comprising an proximal vessel portion, a distal vessel portion, and a vessel narrowing located between the proximal and the distal vessel portion; and determine, from the imaging data, a pressure difference for a first location and a second location in the cardiovascular vessel, the first location and the second location provided in the proximal and the distal vessel portions, respectively. The determining of the absolute pressure difference comprises: determining, from the magnetic resonance imaging data, a preliminary pressure value indicating a pressure difference between the first location and the second location; determining, from the magnetic resonance imaging data, time dependent velocity data for blood flow in the first location and the second location; determining, from the time dependent velocity data, a velocity and time dependent pressure value indicating a velocity and time dependent pressure difference between the first location and the second location; and determining the absolute pressure difference for the first location and the second location by combining the preliminary pressure value and the velocity dependent pressure value.

The determining of the pressure difference comprises: determining, from the imaging data, a preliminary pressure value indicating a pressure difference between the first location and the second location; determining, from the imaging data, time dependent velocity data for blood flow in the first location and the second location; determining, from the time dependent velocity data, a velocity and time dependent pressure value indicating a velocity and time dependent pressure difference between the first location and the second location; and determining the pressure difference for the first location and the second location by combining the preliminary pressure value and the velocity dependent pressure value.

The pressure difference or gradient may be determined as an absolute pressure difference or gradient for the first location and the second location in the cardiovascular vessel.

The determining of the velocity dependent pressure value may comprise applying the momentum Navier-Stokes equation.

The determining of the pressure difference may comprise determining the velocity dependent pressure value according to the following equation:

$$p = \rho \, (\Delta u / \Delta t) dx,$$

wherein dx is the distance along the cardiovascular vessel between first and second location, $\Delta t$ is a time shift between peak blood flow rates in the proximal and the distal vessel portions, and the $\Delta u$ is the change of the mean velocity of blood flow at the distal vessel portion during the time shift. For example, the distance may be a distance between a first and a second location along the aorta.

For the different embodiments, pressure values are determined for three dimensional regions or areas (locations) for which the imaging data provided information. Consequently, the pressure difference is indicating a difference in pressure between the three dimensional regions or areas (locations).

The determining of the preliminary pressure values may comprise determining a relative pressure difference for the first and second location in the time-point when blood flow rate measured in magnetic resonance imaging data is maximum at location one.

The determining of the preliminary pressure values may comprise determining the preliminary pressure values in a time-point when a blood flow rate determined from magnetic resonance imaging data is maximum at the first location.

The combining may comprise adding the supplementary pressure values to the preliminary pressure values for the first and second location.

The providing may comprise providing imaging data indicative of a vessel narrowing assigned to one of the following: aortic coarctation; stenosis in an artery such as coronary artery, carotid artery, intracranial artery, renal artery, hepatic artery, femoral artery, and peripheral artery; and stenotic heart valve such as aortic valve, mitral valve, pulmonary valve, and tricuspid valve.

The providing of the imaging data may comprise providing imaging data selected from the following group: magnetic resonance imaging (MRI) data, echocardiography imaging data, computer tomography (CT) imaging data, and computer simulated fluid dynamics (CFD) imaging data.

The method may be used with the different embodiments for providing the imaging data by applying the principles outlined in the present disclosure. For example, with regard to computer fluid dynamics (CFDs), instead of having measured velocities simulated velocities alongside the three-dimensional vessel are provided (the 3D vessel can be acquired from CT or MRI). From this velocities field both the relative pressure difference and the (absolute) pressure difference are determined. Regarding echocardiography, a 3D structure of the full vessel between the locations may be considered.

The pressure difference which can be determined by the method disclosed may also be referred to as time-shift corrected pressure difference. The technology proposed accounts for a peak pressure time shift between the locations considered.

The exemplary embodiments disclosed above with reference to the method for non-invasively determining a pressure difference in a cardiovascular vessel may apply to the system *mutatis mutandis.*

Description of embodiments

Following further embodiments are described by referring to figures. In the figures, show:

Fig. 1   A block diagram that illustrates the method follow and summarizes the equations used.

Fig. 2   (1) Relative pressure map of the aorta during systole. There are two planes, one in the ascending aorta (location 1, proximal region of the vessel) and other in the descending aorta (location 2, distal region of the vessel). dx is the distance between two planes along the vessel. (2) Flow distribution in the ascending (location 1, blue) and descending (location 2, red) aorta. A and B are the time steps in which there is a peak flow in the ascending and descending aorta, respectively.

Fig. 3   Patients characteristics, pressure gradients based on Riva Rocci (RR), catheterization and Doppler echocardiography and catheterization and time-shift corrected MRI pressure gradients or differences.

Fig. 4   A scatterplot of catheterization peak-to-peak and MRI time-shift corrected pressure gradients or differences in mmHg. The red line is the trend line of the linear correlation between the two methods. The correlation coefficient between both measures (catheter and MRI) is r = 0.95.

Fig. 5   A Bland Altman analysis to assess the agreement between catheterization peak-to-peak pressure gradients and MRI time-shift corrected pressure gradients. The bias is -2.19mmHg and the limit of agreement (2SD) is 3.69 mmHg.

Following, technologies for a non-invasive MRI (Magnetic Resonance Imaging) based analysis are described that take into account the peak pressure time-shift between the ascending and descending aorta. The results of the experimental analysis are compared to peak-to-peak pressure differences or gradients obtained by catheterization.

In a center study twenty-one patients (n=11 male, n=10 female, age range 6 to 52 years, mean age $23\pm15$ years) were enrolled. Inclusion criteria were clinical indication for cardiac catheterization due to CoA based on Doppler gradients and / or arterial hypertension (Baumgartner et al., European Heart Journal, 2010, 31(23): p. 2915-2957). Exclusion criteria were any contraindications for MRI. Institutional research ethics committee following the ethical guidelines of the 1975 Declaration of Helsinki approved the study. Written consent was obtained from the participants and / or their guardians.

MRI examination was performed, in median, 1 day before routine catheterization procedure. MRI was conducted in a 1.5 T magnetic resonance scanner (Achieva R 3.2.2.0, Philips Medical Systems, Best, the Netherlands) using a 5-element cardiac phased-array coil (Philips Medical Systems).

High resolution three-dimensional anatomical (3D Whole Heart MRI sequence) and three directional blood flow velocities ($v_x$, $v_y$, $v_z$) data over the cardiac cycle (4D-VENC MRI sequence) were measured in the thoracic aorta. 3D whole heart MRI sequence was acquired at end-diastole time. The typical scan parameters where: acquisition matrix 100 x 100; Si voxel size $1.2 \times 1.2 \times 2.0$ mm; repetition time 3.6 ms; echo time 1.8 ms. Acquisition duration was typically 2 min. 4D-VENC MRI was acquired using anisotropic segmented k-space with electrocardiographic gating.

Exemplary, 4D-VENC-MRI scan parameters included: field of view feet-to-head 180 mm; anterior-to-posterior 200 to 230 mm (depending on size of the patient); right-to-left 90 to 105 mm (depending on number of slices and slice thickness); acquired voxel size $2.5 \times 2.5 \times 2.5$ mm; reconstruction matrix $128 \times 128$; reconstructed voxel $1.7 \times 1.7 \times 2.5$ mm; flip angle 5°; repetition time 3.5 ms; echo time 2.2 ms; nominal temporal resolution varying with heart rate for 25 time steps; and velocity encoding 400 cm / s. Scan time varied between 8 and 14 min, depending on the chest size.

The aorta segmentation was based on anatomical higher resolution of the 3D Whole Heart sequence. Semi-automatic watershed 3D segmentation of the lumen was performed, using the software ZIBAmira (Zuse Institute Berlin, Berlin, Germany).

To analyze aortic velocity fields, 4D-VENC MRI data was processed in MevisFlow (Fraunhofer MEVIS, Bremen, Germany): Initial anti-aliasing was applied in the presence of phase wrapping. Then, the 3D Whole Heart based segmentation was registered to 4D-VENC MRI data using coarse segmentation of the aorta based on phase contrast. After registration, the anatomical segmentation was used as 3D mask for further Pressure Mapping procedure. From the 3D mask of the aorta, the hemodynamic data (net flow, pressure, velocity and area in each ROI - Region of Interest) was acquired.

To generate the relative pressure maps, Pressure Poisson equation is solved with inhomogeneous Neumann boundary condition using the finite-element method, as suggested by Meier et al. (Meier et al., Computing in Cardiology, 2010):

$$-\Delta p = \nabla.\left(\rho u.\nabla u - \mu \nabla^2 u - \rho g\right) \qquad (2)$$

From the relative pressure maps, the CoA pressure difference is obtained at the instant when the flow rate is maximal in ascending aorta. The equation 2 does not take into account the inertial term that is the first term of the Navier-Stokes equation for the momentum conservation (equation 3):

$$\rho\left(\frac{\partial u}{\partial t}+u.\nabla u\right) = -\nabla p+\nabla.\left(\mu\left(\nabla u+(\nabla u)^T\right)-\frac{2}{3}\mu(\nabla.u)I\right)+F \qquad (3)$$

where u is the velocity, p is the pressure, $\rho$ is the blood flow density ($\rho$ = 1060 kg / m$^3$), $\mu$ is the dynamic viscosity and F the external forces applied to the fluid. This is because this term is zero at the peak flow.

However, the Windkessel function of the distensible aorta causes a pulse pressure propagation with a pulse wave velocity. Thus, a time-shift between both, pressure and volume flow curves in ascending and descending aorta is observed. Consequently, as the fluid acceleration / deceleration in the ascending aorta is zero, the fluid downstream is accelerating or decelerating causing additional positive or negative pressure gradient. To take this into account, we propose a correction of the pressure gradient calculated by equation 2 by using a follow simplified approach:

$$dp_{corr}=-\rho\left(\frac{\Delta u}{\Delta t}\right)dx \qquad (4)$$

where $dx$ is the distance along the vessel between predefined locations in the ascending and the descending aorta, $\Delta t$ is the time shift between peak flow rates in the ascending and the descending aorta and the $\Delta u$ is the change of the mean velocity at the descending aorta site during the time shift (period between peak flow at the ascending and peak flow at the descending aorta).

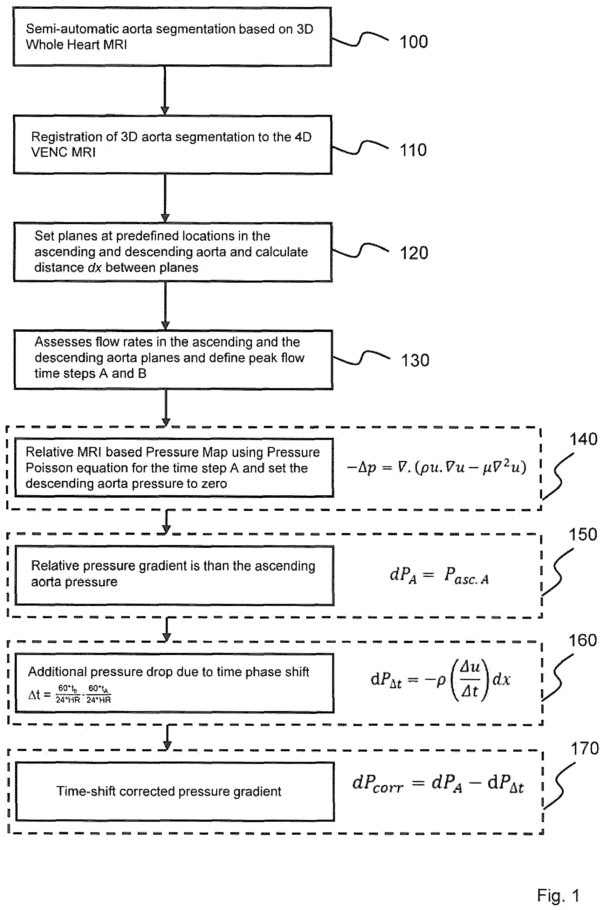

Fig. 1 shows a schematic block diagram for steps which are applied in an exemplary embodiment in a method for non-invasively determining an absolute pressure difference in a cardiovascular vessel.

In a step 100, a vessel segmentation is conducted which may be a semi-automatic vessel segmentation based on 3D Whole Heart MRI (Magnetic Resonance Imaging). In step 110, 3D region comprising the segmented vessel are registered to the 4D VENC MRI.

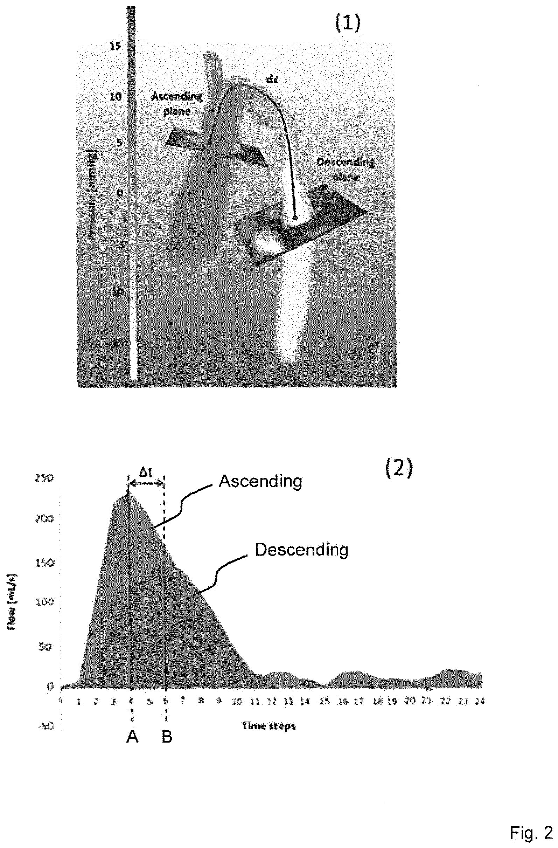

In the embodiment shown, following planes are set at predefined locations in a proximal and a distal aorta, and a distance dx (see Fig. 2) along the vessel between planes is measured (step 120).

In step 130, an assessment of flow rates in the proximal and the distal vessel planes is conducted, and respective peak flow time points are determined (from now on A and B).

A MRI based relative pressure map is determined using Pressure Poisson equation for the time point A (see Fig. 2), and a reference in distal vessel location pressure to zero is set (step 140).

In step 150, a preliminary relative pressure difference is determined which is the proximal vessel relative pressure.

Additional velocity and time dependent pressure difference pressure drop due to time phase shift $\Delta t=B-A$ is determined in step 160.

The time-shift corrected pressure difference is determined in step 170.

Fig. 2 shows (1) Relative pressure map of the aorta during systole. There are two planes, one in the ascending aorta (location1, proximal region of the vessel) and other in the descending aorta (location 2, distal region of the vessel). dx is the distance between two planes along the vessel. Still referring to Fig. 2, it further shows (2) flow distribution in the ascending (location 1, blue) and descending (location 2, red) aorta. A and B are the time steps in which there is a peak flow in the ascending and descending aorta, respectively.

Referring to (1) in Fig. 2, relative pressure distribution / field / map of the aorta during systole is depicted. There are two planes, one in the ascending aorta and other in the descending aorta. dx is the distance between two planes. (2) Flow distribution in the ascending (blue) and descending (red) aorta. A and B are the time steps in which there is a peak flow in the ascending and descending aorta, respectively.

To calculate the time-shift corrected pressure gradient, further requirements must be considered. First, the ROIs have to be cross sectional to the vessel and defined in ascending and descending aorta according measurement sites during catheterization procedure. The ROI in the ascending aorta is placed in the pulmonary trunk plane, whereas the ROI in the descending aorta is placed in the aortic valve plane. The velocity vector field and pressure map must be continuous over the 3D mask.

Routine catheterization procedures aiming to make a final treatment decision were performed under monitoring with a Philips Allura Xper FD 10 / 10 biplane angiography system (Philips Medical Systems, Best, The Netherlands), using contrast agent injection (Ultravist, Schering, Berlin, Germany). The pressure curves were recorded (Schwarzer Haemodynamic Analysing System, Heilbronn, Germany) in two predefined locations in the aorta via catheter pull back. Peak-to-peak pressure gradients across CoA were obtained from these pressure curves and used to validate MRI based pressure mapping.

The analysis of the data was performed with SPSS version 21 (IBM Corporation, Armonk, USA). Data is expressed as mean $\pm$ standard deviation (SD). Effects have been considered significant at $p < 0.05$. The Pearson correlation coefficients and linear regression have been determined. The agreement between catheterization and MRI time-shift corrected pressure gradient measurements was determined with the Bland-Altman analysis. In addition, a two one-sided test (TOST) procedure was used to test equivalency. In this procedure, it is assumed that the population means differ - Null hypothesis - and the goal is to prove the population means equivalency - Alternative hypothesis.

To have an acceptable statistical power of 80%, a minimal sample size of seven patients was necessary to compare both catheter and MRI measurements. The effect size was calculated based on SD of pressure gradients measured with a catheter (4.8 mmHg). The power test was performed for the T-test (differences between two dependent means - matched pairs). The power analysis were performed using G*Power 3.1.9 (Franz Faul, Kiel University, Kiel, Germany).

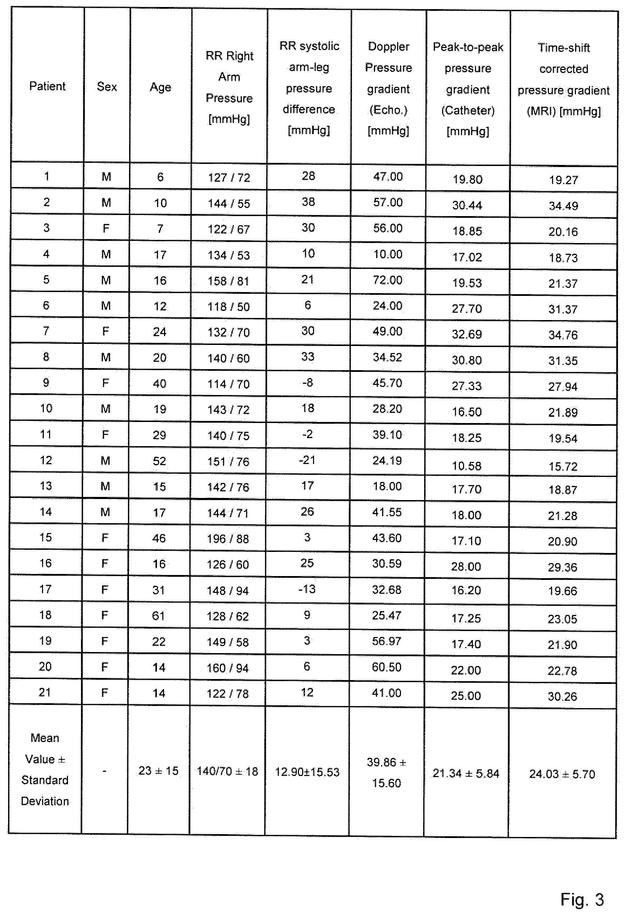

Patient characteristics, hemodynamic baseline measurements and MRI measurements are given in Fig. 3.

TOST statistical analysis (p<0.01) confirms the equivalence between both methods. Simplified Bernoulli via Doppler echocardiography pressure gradients (39.86 $\pm$ 15.60 mmHg) overestimates catheterization measurements, while MRI based relative pressure gradients (15.46 $\pm$ 7.64 mmHg) underestimates.

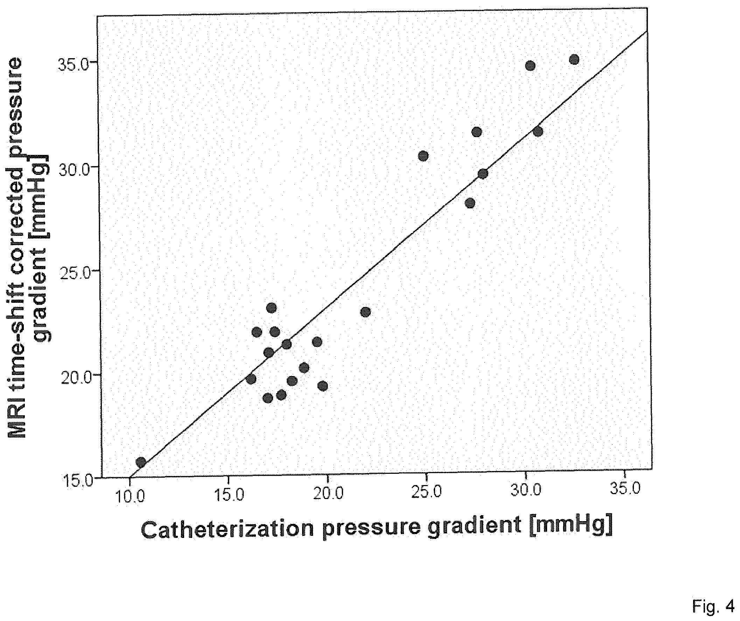

The correlation coefficient between MRI time-shift corrected pressure gradients and catheterization peak-to-peak pressure gradients is 0.95 (Fig. 4).

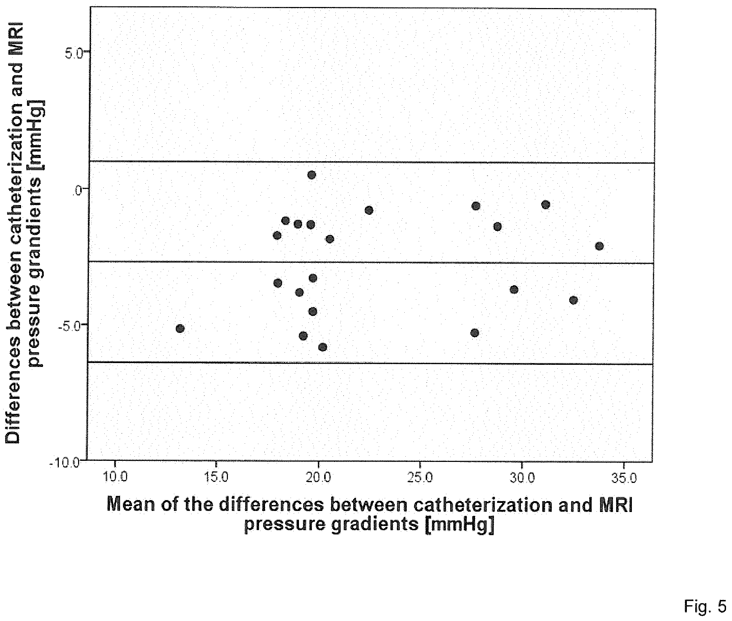

The Bland-Altman analysis shows good agreement between catheterization (21.34 $\pm$ 5.84 mmHg) and MRI time-shift corrected pressure gradients (24.03 $\pm$ 5.70 mmHg). The bias (mean of differences) is -2.19 mmHg and the limit of agreement is 3.69mmHg (Fig. 5).

The ACC / AHA and ESC guidelines recommend intervention for CoA or re-CoA in the presence of upper limp hypertension (>140 / 90 mmHg) and if the pressure gradient exceeds 20 mmHg. Just the method to measure CoA pressure gradient varies. While the ACC / AHA suggest decision making based on invasive peak-to-peak pressure gradient from catheterization and the ESC propose to measure the pressure differences between upper and lower limbs non-invasively (Baumgartner et al., European Heart Journal, 2010, 31(23): p. 2915-2957) (Warnes et al., J Am Coll cardiol, 2008, 52(23): p. e143-263). Both guidelines further suggest intervention when CoA narrowing is severe with a reduction of over 50% of the vessel diameter in hypertensive patients, regardless of pressure gradient.

These guidelines point out the medical need for a non-invasive and an accurate method to measure pressure gradients across CoA. However, currently available tools are not always reliable. RR method is established as a valid method to measure arterial hypertension. However, RR measurements of pressure differences between peripheral arteries are affected by anatomic variation, pulse wave velocities and measurement set-up (e.g. cuff-size). Doppler is widely accessible and the first choice method for diagnostic workup of patients with CoA. Doppler pressure gradient is obtained from simplified Bernoulli equation. This method can lead to pressure gradient overestimation, due to increased arterial stiffness or underestimation in complex anatomy cases, due to field of view limitations.

MRI-derived methods that allow the computation of 3D pressure maps relative to a reference location at all time-points were proposed (Tyszka et al., Journal of Magnetic Resonance Imaging, 2000, 12(2): p. 321-329). Nonetheless, the relative pressure maps do not take into consideration the peak pressure time-shift along the aorta. If the time-shift, due to the Windkessel function, is neglected, the pressure variation that occurs in this interval is not weighed. Therefore, systematic errors in pressure gradient acquisition are induced and relative pressure maps tend to underestimate the pressure gradient across CoA.

The technologies proposed here allow non-invasive measurement of time-shift corrected pressure gradients in patients with vessel narrowing, using the relative pressure gradient and pressure variation over time in the proximal and distal vessel regions, for example in the aorta

Concerning MRI data processing, the Pressure Poisson equation was applied with inhomogeneous Neumann boundary conditions to generate the 3D relative pressure maps. Numerical inconsistencies can occur when using Pressure Poisson equation. To avoid such, there is a vessel size reduction of 5%. The vessel size reduction is considered not critical, since it is not the objective of the method disclosed to analyze the pressure conditions near the vessel wall (Meier et al., Computing Cardiology, 2010, IEEE).

The study was performed in a cohort of CoA 21 patients accessed under typical clinical setting conditions. The study group presented moderate pressure gradients (21.34 $\pm$ 5.84 mmHg), which represents the borderline cases for CoA intervention. Regarding well established methods, the results confirm the respective known limitations. Even though, all the patients had catheterization indication, arterial hypertension was present in 57.1% of the cases. An catheterization derived pressure gradient exceeding 20 mmHg was present in 30% of the patients. Furthermore n=3 were neither hypertensive nor had a catheterization pressure gradient exceeding 20 mmHg.

The proposed technologies improve options for non-invasive diagnostic with CoA.

The features disclosed in this specification, the figures and / or the claims may be material for the realization of various embodiments, taken in isolation or in various combinations thereof.

**Claims**

1.  Method for non-invasively determining a pressure difference in a cardiovascular vessel, comprising

    - providing imaging data indicative of imaging signals detected in a region of interest of a cardiovascular vessel, the region comprising a proximal vessel portion, a distal vessel portion, and a vessel narrowing located between the proximal and the distal vessel; and
    - determining, from the imaging data, a pressure difference between a first location and a second location in the cardiovascular vessel portion, the first location and the second location provided in the proximal and the distal vessel portions, respectively;

    wherein the determining the pressure difference comprises

    - determining, from the imaging data, a preliminary pressure value indicating pressure difference between the first location and the second location;
    - determining, from the imaging data, time dependent velocity data for blood flow in the first location and the second location;
    - determining, from the time dependent velocity data, a velocity and time dependent pressure value indicating a velocity and time dependent pressure difference between the first location and the second location; and
    - determining the pressure difference for the first location and the second location by combining the preliminary pressure value and the velocity dependent pressure value.

2.  Method of claim 1, wherein the determining of the velocity dependent pressure value comprises applying the Navier-Stokes momentum equation.

3.  Method of claim 2, wherein the determining of the pressure difference comprises determining the velocity dependent pressure value according to the following equation:

$$p = \rho \left( \frac{\Delta u}{\Delta t} \right) dx,$$

    wherein dx is the distance along the cardiovascular vessel between first and second location, $\Delta t$ is a time shift between peak blood flow rates in the proximal and the distal vessel portions, and the $\Delta u$ is the change of the mean velocity of blood flow at the distal vessel portion during the time shift.

4.  Method of at least one of the preceding claims, wherein the determining of the preliminary pressure values comprises determining a relative pressure difference for the first and second location.

5.  Method of at least one of the preceding claims, wherein the determining of the preliminary pressure values comprises determining the preliminary pressure values in a time-point when a blood flow rate determined from magnetic resonance imaging data is maximum at the first location.

6.  Method of at least one of the preceding claims, wherein the combining comprises adding the supplementary pressure values to the preliminary pressure values for the first and second location.

7.  Method of at least one of the preceding claims, wherein the providing comprises providing imaging data indicative of a vessel narrowing assigned to one of the following: aortic coarctation; stenosis in an artery such as coronary artery, carotid artery, intracranial artery, renal artery, hepatic artery, femoral artery, and peripheral artery; and stenotic heart valve such as aortic valve, mitral valve, pulmonary valve, and tricuspid valve.

8.  Method of at least one of the preceding claims, wherein providing the imaging data comprises providing imaging data selected from the following group: magnetic resonance imaging data, echocardiography imaging data, computer tomography imaging data, and computer simulated fluid dynamics imaging data.

9.  System for non-invasively determining a pressure difference in a cardiovascular vessel, comprising one or more processors configured to

- receive imaging data indicative of imaging signals detected in a region of interest of a cardiovascular vessel, the region comprising a proximal vessel portion, a distal vessel portion, and a vessel narrowing located between the proximal and the distal vessel portion; and
- determine, from the imaging data, a pressure difference for a first location and a second location in the cardiovascular vessel, the first location and the second location provided in the proximal and the distal vessel portions, respectively;

wherein the determining the pressure difference comprises

- determining, from the imaging data, a preliminary pressure value indicating pressure difference between the first location and the second location;
- determining, from the imaging data, time dependent velocity data for blood flow in the first location and the second location;
- determining, from the time dependent velocity data, a velocity and time dependent pressure value indicating a velocity and time dependent pressure difference between the first location and the second location; and
- determining the pressure difference for the first location and the second location by combining the preliminary pressure value and the velocity dependent pressure value.

Semi-automatic aorta segmentation based on 3D Whole Heart MRI ⟶ 100

Registration of 3D aorta segmentation to the 4D VENC MRI ⟶ 110

Set planes at predefined locations in the ascending and descending aorta and calculate distance $dx$ between planes ⟶ 120

Assesses flow rates in the ascending and the descending aorta planes and define peak flow time steps A and B ⟶ 130

Relative MRI based Pressure Map using Pressure Poisson equation for the time step A and set the descending aorta pressure to zero

$$-\Delta p = \nabla . (\rho u . \nabla u - \mu \nabla^2 u)$$ ⟶ 140

Relative pressure gradient is than the ascending aorta pressure

$$dP_A = P_{asc.A}$$ ⟶ 150

Additional pressure drop due to time phase shift

$$\Delta t = \frac{60 \cdot t_b}{24 \cdot HR} - \frac{60 \cdot t_A}{24 \cdot HR}$$

$$dP_{\Delta t} = -\rho \left(\frac{\Delta u}{\Delta t}\right) dx$$ ⟶ 160

Time-shift corrected pressure gradient

$$dP_{corr} = dP_A - dP_{\Delta t}$$ ⟶ 170

Fig. 1

Fig. 2

| Patient | Sex | Age | RR Right Arm Pressure [mmHg] | RR systolic arm-leg pressure difference [mmHg] | Doppler Pressure gradient (Echo.) [mmHg] | Peak-to-peak pressure gradient (Catheter) [mmHg] | Time-shift corrected pressure gradient (MRI) [mmHg] |
|---|---|---|---|---|---|---|---|
| 1 | M | 6 | 127 / 72 | 28 | 47.00 | 19.80 | 19.27 |
| 2 | M | 10 | 144 / 55 | 38 | 57.00 | 30.44 | 34.49 |
| 3 | F | 7 | 122 / 67 | 30 | 56.00 | 18.85 | 20.16 |
| 4 | M | 17 | 134 / 53 | 10 | 10.00 | 17.02 | 18.73 |
| 5 | M | 16 | 158 / 81 | 21 | 72.00 | 19.53 | 21.37 |
| 6 | M | 12 | 118 / 50 | 6 | 24.00 | 27.70 | 31.37 |
| 7 | F | 24 | 132 / 70 | 30 | 49.00 | 32.69 | 34.76 |
| 8 | M | 20 | 140 / 60 | 33 | 34.52 | 30.80 | 31.35 |
| 9 | F | 40 | 114 / 70 | -8 | 45.70 | 27.33 | 27.94 |
| 10 | M | 19 | 143 / 72 | 18 | 28.20 | 16.50 | 21.89 |
| 11 | F | 29 | 140 / 75 | -2 | 39.10 | 18.25 | 19.54 |
| 12 | M | 52 | 151 / 76 | -21 | 24.19 | 10.58 | 15.72 |
| 13 | M | 15 | 142 / 76 | 17 | 18.00 | 17.70 | 18.87 |
| 14 | M | 17 | 144 / 71 | 26 | 41.55 | 18.00 | 21.28 |
| 15 | F | 46 | 196 / 88 | 3 | 43.60 | 17.10 | 20.90 |
| 16 | F | 16 | 126 / 60 | 25 | 30.59 | 28.00 | 29.36 |
| 17 | F | 31 | 148 / 94 | -13 | 32.68 | 16.20 | 19.66 |
| 18 | F | 61 | 128 / 62 | 9 | 25.47 | 17.25 | 23.05 |
| 19 | F | 22 | 149 / 58 | 3 | 56.97 | 17.40 | 21.90 |
| 20 | F | 14 | 160 / 94 | 6 | 60.50 | 22.00 | 22.78 |
| 21 | F | 14 | 122 / 78 | 12 | 41.00 | 25.00 | 30.26 |
| Mean Value ± Standard Deviation | - | 23 ± 15 | 140/70 ± 18 | 12.90±15.53 | 39.86 ± 15.60 | 21.34 ± 5.84 | 24.03 ± 5.70 |

Fig. 3

Fig. 4

Fig. 5

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MIRZAEE, HANIEH AND HENNERMUTH, ANJA: "NONINVASIVE MEASUREMENT OF INTRAVASCULAR PRESSURE GRADIENTS BASED ON 3D ANATOMY AND 4DFLOW IMAGE FUSION", INTERNATIONAL SOCIETY FOR MAGNETIC RESONANCE IN MEDICINE, ISMRM, 2030 ADDISON STREET, 7TH FLOOR, BERKELEY, CA 94704 USA, vol. 23, 15 May 2015 (2015-05-15), page 2753, XP040668430, | 1-4,6-9 | INV. A61B5/02 A61B5/021 A61B5/026 G06T7/00 ADD. A61B5/055 |
| Y | * the whole document * | 5 | |
| Y | US 2013/243294 A1 (RALOVICH KRISTOF [DE] ET AL) 19 September 2013 (2013-09-19) | 5 | |
| A | * paragraph [0033] - paragraph [0049] * * figures 2-10 * | 1-4,6-9 | |
| A | H L BERGMAN ET AL: "Pressure Gradients Across Aortic Coarctations: A Comparison of Magnetic Resonance Imaging to Doppler Ultrasound", PROCEEDINGS OF THE INTERNATIONAL SOCIETY FOR MAGNETIC RESONANCE IN MEDICINE, vol. 1996, 1 January 1996 (1996-01-01), page 1374, XP055459150, DOI: 10.1002/mrmp.22419960128 * the whole document * | 1-9 | |

| | |
|---|---|
| | TECHNICAL FIELDS SEARCHED (IPC) |
| | A61B G06T |

-/--

The present search report has been drawn up for all claims

| Place of search | Date of completion of the search | Examiner |
|---|---|---|
| The Hague | 14 March 2018 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 17 19 1637

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SEBASTIAN MEIER ET AL: "A Fast and Noise-Robust Method for Computation of Intravascular Pressure Difference Maps from 4D PC-MRI Data", 5 October 2012 (2012-10-05), STATISTICAL ATLASES AND COMPUTATIONAL MODELS OF THE HEART. IMAGING AND MODELLING CHALLENGES, SPRINGER BERLIN HEIDELBERG, BERLIN, HEIDELBERG, PAGE(S) 215 - 224, XP047027340, ISBN: 978-3-642-36960-5 * page 216, paragraph 4 - page 222, paragraph 2 * | 1-9 | |

-----

|  | TECHNICAL FIELDS SEARCHED (IPC) |
|---|---|

The present search report has been drawn up for all claims

| Place of search | Date of completion of the search | Examiner |
|---|---|---|
| The Hague | 14 March 2018 | Görlach, Tobias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 1637

This annex lists the patent family members relating to the patent documents cited in  the above-mentioned European search report.
The members are as contained in the European Patent Office EDP file on
The European Patent Office is in no way liable for these particulars which are merely given for the purpose of information.

14-03-2018

| Patent document cited in search report | Publication date | Patent family member(s) | Publication date |
|---|---|---|---|
| US 2013243294    A1 | 19-09-2013 | NONE | |

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BAUMGARTNER et al.** *European Heart Journal,* 2010, vol. 31 (23), 2915-2957
- **WARNES et al.** *J Am Coll cardiol,* 2008, vol. 52 (23), e143-263
- **TYSZKA et al.** *Journal of Magnetic Resonance Imaging,* 2000, vol. 12 (2), 321-329
- **RIESENKAMPFF et al.** *JACC: Cardiovascular Imaging,* 2014, vol. 7 (9), 920-926
- **MEIER et al.** *Computing in Cardiology,* 2010
- **MEIER et al.** Computing Cardiology. IEEE, 2010